# EUROPÄISCHE PATENTSCHRIFT

(11) **EP 0 952 856 B1**
(45) Veröffentlichungstag und Bekanntmachung des Hinweises auf die Patenterteilung: **03.12.2003**
(21) Anmeldenummer: 97945714.0
(22) Anmeldetag: 10.12.1997
(51) Int. Cl.: A61M 1/06

(54) **MUTTERMILCHPUMPE UND VENTIL HIERFÜR**
BREAST PUMP AND BREAST PUMP VALVE
TIRE-LAIT ET VALVE DE TIRE-LAIT

(30) Priorität: 16.12.1996 DE 19652232
(43) Veröffentlichungstag der Anmeldung: 03.11.1999
(73) Patentinhaber: NUESCH, LOGISTIK, 9524 ZUZWIL (CH)
(72) Erfinder: NÜESCH, Heinrich, CH-9524 Zuzwil (CH)
(74) Vertreter: Révy von Belvárd, Peter
(86) Internationale Anmeldenummer: CH9700460
(87) Internationale Veröffentlichungsnummer: WO98026817

(56) Entgegenhaltungen:
- EP-A- 0 198 651
- WO-A-96/24791
- FR-A- 2 673 260
- US-A- 4 673 388
- US-A- 4 857 051
- US-A- 4 886 494

## Beschreibung

Die Erfindung bezieht sich auf eine Muttermilchpumpe nach dem Oberbegriff des Anspruches 1, sowie auf ein Ventil für eine solche Muttermilchpumpe mit den Oberbegriffsmerkmalen des Anspruches 4.

Am Markt waren Muttermilchpumpen mit einem Rückschlagventil, das im Anschluß an den Ventilkörper, in einer gegen den Milchsammler zu gelegenen Vorkammer ein durch diese Vorkammer hindurch frei bewegliche Klappe besaß. Die Vorkammer war also nur durch die Klappe gegen den Milchsammler abgeschlossen, wobei diese Klappe bei Freigabe von peripheren Öffnungen von einwärts gebogenen, die Verbindungseinrichtung bildenden Lappen der Kammerwandung gehalten wurde. Eine solche Ausbildung ist aber schwer zu reinigen und genügte deshalb den Hygieneanforderungen nicht. Zudem war die Klappe zentrisch innerhalb der Kammer praktisch ungeführt, so daß sie sich gegebenenfalls querzustellen vermochte und dann ein einwandfreier Betrieb nicht mehr gewährleistet war.

Nun sind Muttermilchpumpen beispielsweise aus der US-A-4,929,229 bekannt geworden, bei denen eine einfache Steckverbindung für die Klappe in der Mitte der Stirnfläche des Ventils vorgesehen war, das so leicht abgenommen und gereinigt werden konnte. Ein Problem dabei ist, daß zwar die Milch im biologischen Rhythmus eines saugenden Babys abgesaugt wird, sich dadurch aber sehr stark unterschiedliche Milchmengen ergeben, die dem Milchsammler, meist in Form eines angeschlossenen Behälters, zugeführt werden müssen. Dies hat in der Vergangenheit zu verschiedenen Unzukömmlichkeiten im Betrieb einer solchen Muttermilchpumpe geführt. Bei großen Milchmengen konnte nämlich die Milch die Pumpeneinheit erreichen, die dabei leicht zerstört, mindestens aber für eine Zeit lang bis zur Reparatur bzw. Zerlegung und Reinigung unbrauchbar gemacht wurde.

Eine Muttermilchpumpe mit den Merkmalen des Oberbegriffes des Oberbegriffes des Anspruches 1 ist aus der EP-A-0 198 651 bekannt geworden. Auch hier stellt aber die unbedingt nötige Reinigung ein Problem dar.

Untersuchungen der Anmelderin haben gezeigt, daß das zuvor genannte Problem verschiedene Ursachen hatte, die beim Stande der Technik sogar synergetisch, im verschlechternden Sinne, zusammenwirkten. Daher ist es Ziel der Erfindung, eine Muttermilchpumpe der eingangs genannten Art so auszubilden, daß die Reinigung des Ventilkörpers leichter erfolgen kann, und dies gelingt durch das Kennzeichen des Anspruches 1.

Zunächst wurde erkannt, daß das Ventil nach der US-A-4,929,229 zwar die Hygienefrage weitgehend löste, aber durch seine zentrische Verbindungseinrichtung nur eine verringerte Elastizität der Klappe zuließ. Zwar bestand ein gewisses Vorurteil der Fachwelt, daß nur durch eine zentrische Verbindungseinrichtung eine gleichmäßige Abdeckung der im Stande der Technik vorgesehenen Mahrzahl von Durchlaßöffnungen zu erzielen war. Dieses Vorurteil erwies sich jedoch nach den Untersuchungen der Anmelderin als unbegründet. Umgekehrt wird durch die nach dem Kennzeichen des Anspruches 1 vorgesehene, am Rande der Stirnseite, d.h. exzentrisch, angelenkte Klappe dieser eine erhöhte Flexibilität verliehen. Es wurde nämlich erkannt, daß bei einer zentrischen Anordnung der Verbindungseinrichtung die Klappe nur jeweils einen freien Radius besitzt, entlang dem sie ihre Flexibilität entfalten kann. Dadurch wurde die Klappe aber relativ steif und stellte daher auch bei Freigabe der Durchlaßöffnung einen nicht unbeträchtlichen Strömungswiderstand dar. Durch die erfindungsgemäße Ausbildung wird hingegen die freie biegsame Länge der Klappe vergrößert und die Klappe erhält Eigenschaften einer größeren Schmiegsamkeit und Elastizität, so daß sie sich unter Sogwirkung dicht an die jeweilige Durchlaßöffnung anlegt, aber unter der Last auch nur einer geringen Menge von Milch die Durchlaßöffnung praktisch widerstandsfrei freigibt. Durch die Anordnung des Befestigungsflansches ist anderseits das Ventil leicht abzunehmen und zu reinigen.

Bevorzugt sind jedoch die Merkmale des Anspruches 2 vorgesehen, wobei die Reinigung durch Abnahme und das neuerliche Aufstecken des Befestigungsflansches des Ventilkörpers leicht erfolgen kann, gleichzeitig aber die Verbindungseinrichtung keinen nachteiligen Effekt auf das Scharnier auszuüben vermag.

Gerade dann, wenn die Klappe, entsprechend der genannten erfindungsgemäßen Ausbildung, eine erhöhte Elastizität und Schmiegsamkeit besitzt, kann eine einzige Durchlassöffnung nicht beliebig groß gemacht werden, weil sich die weiche Klappe sonst beim Saughub der Pumpeneinheit in ihr verfangen kann. Deshalb ist die Ausbildung nach Anspruch 3, Merkmal b) bevorzugt.

Ferner ist es bevorzugt, wenn das Merkmal a) des Anspruches 3 verwirklicht ist, weil damit die Lage des abnehmbaren Ventils an der Durchlassöffnung in Axialrichtung bestimmt wird, so dass die Klappe nicht durch zu tiefes Aufstecken oder zu wenig tiefes Aufstecken in ihrer Funktion behindert wird. Die endliche, die Klappe eng umschließende Dicke des Randes bewirkt dabei eine Abdichtung zwischen ihm und der Klappe, die somit beim Saughub rasch in die so gebildete Öffnung des Randes hineingesogen wird. Der Rand kann dabei gegebenenfalls rund um die Klappe verdickt sein, doch ist dies im allgemeinen nicht erforderlich.

An sich könnte ja das Scharnier auf die verschiedenste Weise ausgebildet werden. Durch die Ausbildung nach Anspruch 3, Merkmal c) ergibt sich jedoch ein glattes, und damit leicht zu reinigendes, Kunststoffscharnier, das auch leicht in einem Stück mit der Klappe herstellbar ist.

All diese Merkmale werden bzw. können natürlich in einem erfindungsgemässen Ventil für eine solche Muttermilchpumpe verwirklicht sein.

Weitere Einzelheiten, Merkmale und Vorteile der Erfindung ergeben sich an Hand der nachfolgenden Beschreibung eines in der Zeichnung schematisch dargestellten bevorzugten Ausführungsbeispieles, bei welchem:
- Fig. 1: einen Längsschnitt durch Brustkörper, Kanalsystem und Ventil einer erfindungsgemäßen Muttermilchpumpe zeigt, wogegen
- Fig. 2: das erfindungsgemäße Ventil in Perspektivansicht veranschaulicht;
- Fig. 3: eine Detailansicht aus Fig. 1 in größerem Maßstab zur Veranschaulichung eines erfindungsgemäßen Verschlußsystems für die Einstellung der Zufuhr von Falschluft in Geschlossenlage und in
- Fig. 4: in teilweise geöffnetem Zustande ist, wogegen die
- Fig. 5: den Deckel und den Nabenteil dieses Systems in einer Perspektivansicht darstellt.

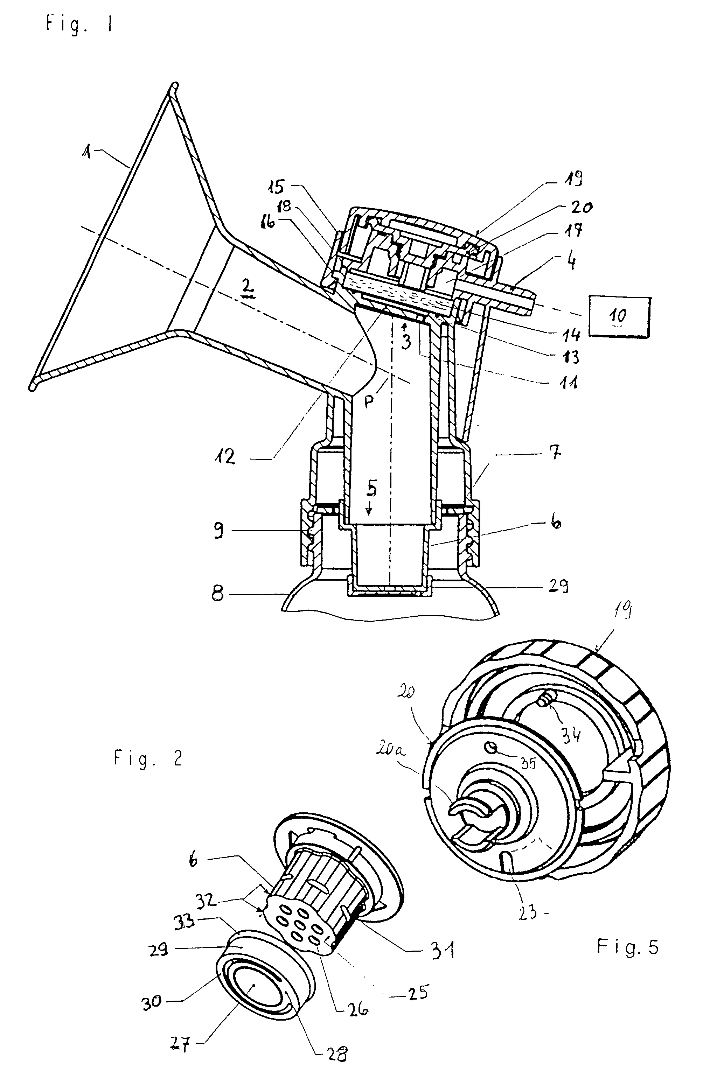

In Fig. 1 ist ein Längsschnitt des Oberteils einer Muttermilchpumpe gezeigt, die einen die Brust einer Frau aufnehmenden Brustkörper 1 aufweist. An den Brustkörper 1 ist ein Abflußkanal 2 mit abwärts gerichteter Neigung angeschlossen, so daß das Abfließen der abgepumpten Milch erleichtert wird. Dieser Abflußkanal 2 verzweigt sich an einem Verzweigungspunkte P. Ein erster Abzweigkanal 3 ist aufwärts gerichtet und führt über einen mit ihm einen Winkel einschließenden ersten Anschlußstutzen 4 zu einer lediglich angedeuteten, an sich bekannten Pumpeneinheit 10. Ein abwärts führender Abzweigkanal 5 mündet in einen an ihm abnehmbar aufgesetzten Ventilkörper 6, der von einem Abdeckring 7 umgeben ist. Unter dem Ventil 6 ist ein Milchsammelbehälter 8 vorgesehen, der mittels eines Gewindes 9 an den Oberteil der Muttermilchpumpe anschließbar ist.

Selbst wenn also eine große Milchmenge abgesaugt würde, so daß die Durchlaßkapazität am Ventilkörper 6 nicht ausreichte, um diese Menge rasch in den Miclsammler 8 (der auch anders ausgebildet sein könnte) abzuführen, so würde das Niveau der Milch zunächst bis zur Höhe des Abflußkanales 2 ansteigen, wo ein gegenüber dem Abzweigkanal 5 erweitertes Volumen zur Aufnahme der Milchmenge zur Verfügung steht. Den aufwärts gerichteten Abzweigkanal 3 und die über den Stutzen angeschlossene Pumpeneinheit 10 könnte also diese Milch kaum erreichen.

Zur Sicherheit aber weist der aufwärts gerichtete Abzweigkanal 3, 4 auch noch nach dem Punkte P der Einmündung des ersten Abzweigkanalabschnittes 3 in den Abflußkanal 2 in Richtung zur Pumpeneinheit 10 hin einen Strömungswiderstand auf, der zunächst durch eine verengte Durchtrittsöffnung 11 verringerten Querschnittes in einer Querwand 12 gegeben ist. Es versteht sich, daß ein solcher Strömungswiderstand auch an jedem anderen Ort entlang der zur Pumpeneinheit 10 führenden Leitung angebracht sein könnte, daß aber die Anbringung im aufwärts gerichteten Abzweigkanalabschnitt 3 bevorzugt ist.

Hinter der Durchtrittsöffnung 11, gesehen in Richtung zur Pumpeneinheit 10, befindet sich eine kleine Vorkammer 13, die durch einen weiteren Strömungswiderstand in Form eines Filters 14 abgedeckt ist. Dieses Filter 14 besteht zweckmäßig aus einem Textilmaterial, wie es für Regenmäntel bekannt geworden ist, um dort den Durchtritt von Luft zu gestatten, im Falle eines Regens aber unter dem Einfluß der Nässe die Poren schließt. Hier wird dieses Material dazu verwendet, die Saugwirkung der Pumpeneinheit 10 an sich nicht zu behindern, für den auf Grund der bereits beschriebenen Maßnahmen jedoch unwahrscheinlichen Fall eines Durchtrittes von Milch in die Vorkammer 13 für diese eine undurchdringliche Barriere zu bilden.

Um das Auswechseln des Filters 14 zu gestatten, ist ein an den ersten Abzweigkanal 3 anschließender, insbesondere abnehmbarer Stutzen 15 vorgesehen, der am Abzweigkanal 3 mittels eines Bajonettverschlusses 16 befestigt ist. Ferner befindet sich hinter dem Filter 14 eine weitere Kammer 17, die mit dem relativ dünnen Anschlußstutzen 4 verbunden ist, so daß selbst bei einer Fehlfunktion des Filters 14 oder bei nicht eingesetztem Filter 14 noch ausreichend Raum 13, 17 zur etwa eingedrungener Milch vorhanden ist. Ein Positionierelement 18 hält das Filter 14 in seiner Lage, so daß es sich nicht unter Sogwirkung aufwölben kann.

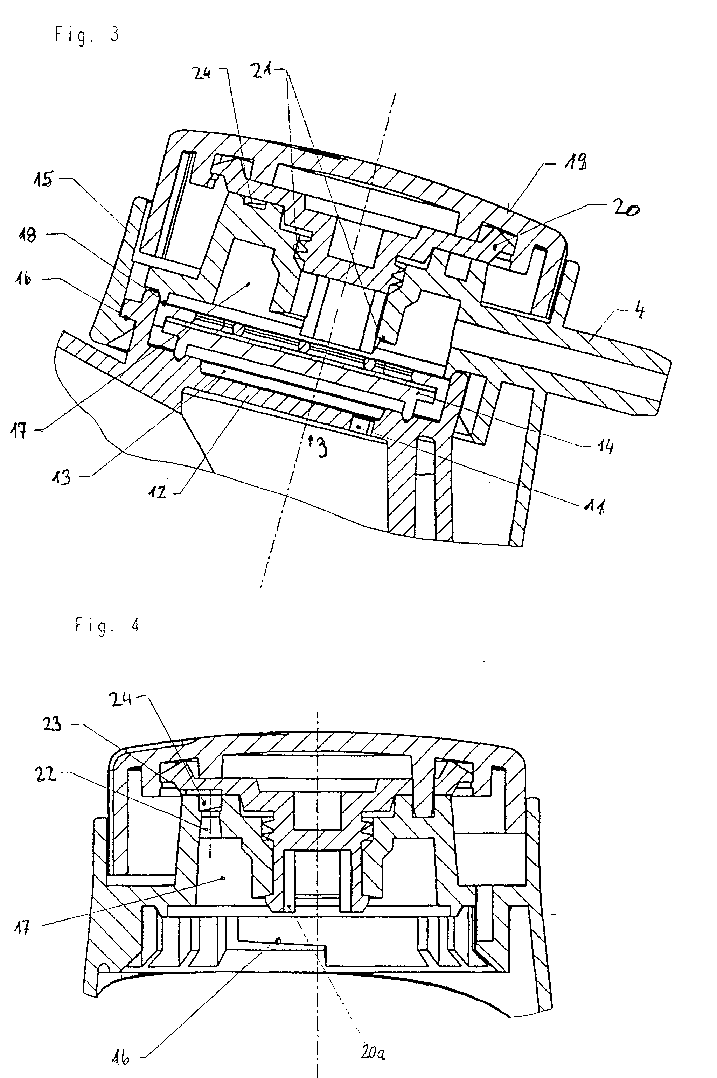

Eine weitere Maßnahme zur Verhinderung des Eindringens von Milch in die Pumpeneinheit 10 besteht darin, daß die auf den Abflußkanal 2 und den Brustkörper 1 wirkende effektive Saugleistung der Pumpeneinheit durch die Möglichkeit der Zuführung von Falschluft begrenzt wird. Wie besonders aus Fig. 3 hervorgeht, ist zu diesem Zweck der Stutzen 15 durch einen Deckel 19 abgeschlossen, der mit einem Nabenteil 20 verbunden ist. Der Nabenteil 20 sitzt in einem mittigen Lager 21, das einteilig oder gesondert am Stutzen 15 ausgebildet ist. Zweckmäßig ist ein Schnappverbindungsteil 20a zur Schaffung einer festen Verbindung mit dem Stutzen 15. Auf diese Weise ist der Deckel 19 über den Nabenteil 20 drehbar am Stutzen 15 befestigt. Die Fig. 5 veranschaulicht eine formschlüssige Verbindung zwischen dem Deckel 19 und dem Nabenteil 20, die an sich in beliebiger Weise ausgebildet sein könnte, in der dargestellten einfachen Form aber derart gestaltet ist, daß mindestens ein parallel zur Drehachse erstreckender Vorsprung (Stift 34) und eine damit verzahnende Vertiefung (Loch 35) ineinander eingreifen.

Der Stutzen 15 besitzt einen zentrischen Zylinderring 24 mit über seinen Umfang sich verändernder Tiefe und mit einer in die Kammer 17 führenden Öffnung 22, die mit einer Falschluftöffnung 23 (Fig. 4) des Stutzens 15 mehr oder weniger zur Deckung gebracht werden kann. Fig. 5 zeigt die in diesem Ausführungsbeispiel kanalartig ausgebildete Öffnung 23, doch kann sie alternativ sich entlang der strichlierten Linie keilförmig erstrekken, um ihren Querschnitt zu verändern.

Dadurch ist die effektive Saugleistung der Pumpeneinheit 10 durch Verschließen der Falschluftöffnung 23 oder mehr oder weniger starkes Öffnen vorzugsweise einstellbar. So zeigt Fig. 4 die kanalartige Falschluftöffnung 23, die gegebenenfalls keilförmig verlaufen kann, um den Strömungswiderstand zu verändern. Es versteht sich, daß diese Verstellhilfe nicht unbedingt vorgesehen zu sein braucht, da eine Falschluftöffnung unveränderlichen Querschnitts gegebenenfalls auch von Hand aus, ähnlich den Löchern einer Blockflöte, abgedeckt werden könnte. Auch könnte die Falschluftöffnung 23 an jedem beliebigen Ort innerhalb der vom Anschlußkanal 2 zur Pumpeneinheit 10 führenden Leitung vorgesehen werden, doch ist die Anordnung hinter dem Strömungswiderstand 11, 14 verständlicherweise besonders zweckmäßig.

Wie besonders aus Fig. 2 ersichtlich ist, ist der Ventilkörper 6 an seiner Stirnseite 25 mit einer Mehrzahl von Durchlaßöffnungen 26 für die Muttermilch versehen. Dies sichert einen relativ großen Durchlaßquerschnitt für die Milch, ohne daß die einzelne Öffnung 26 zu groß gemacht werden müßte. Zwar ist diese Maßnahme an sich bekannt, erfolgte aber bisher aus anderen Gründen. Hier hängt dies mit der besonderen Ausbildung einer Rückschlagklappe 27 zusammen, die nur über ein am Rande der Stirnseite 25 angeordnetes Scharnier 28 sowie über einen daran angeschlossenen Ringflansch 29 mit dem Ventilkörper 6 verbunden werden kann. Wie ersichtlich, ist die Klappe 27 mit dem Scharnier 28 aus schmiegsamem Material nach Art eines Kunststoffscharniers einteilig ausgebildet, so daß sich eine flache, nach Abnahme leicht zu reinigende Ausgestaltung ergibt.

Durch diese exzentrische Anlenkung der Ventilklappe 27 ergibt sich ein relativ großer, sich über den ganzen Durchmesser der Klappe 27 erstreckender freier Abbiegebereich, auf den also unter der Last der in den Ventilkörper 6 gelangten Milch ein relativ großes Drehmoment wirkt, so daß sich eine solche Klappe leicht zur vollen Freigabe der Öffnungen 26 abbiegen kann. Dies wird zusätzlich dadurch begünstigt, daß die große Abbiegelänge der Klappe 27 ihr eine besondere Biegsamkeit verleiht. Es ist klar, daß der Durchmesser der Klappe 27 etwa so weit reichen muß, daß der Außendurchmesser der Reihe der Öffnungen 26 abgedeckt ist. Alternativ könnte eine Falschluftzufuhr durch Freilassen eines Flächenbereiches der radial äußeren Öffnungen 26 erzielt werden, obwohl dies aus den oben angeführten Gründen nicht bevorzugt ist.

Mit dem Ringflansch 29 ist ein peripherer, das Scharnier 28 mit bildender Rand 30 verbunden, der eine endliche Dicke in Axialrichtung des Ventilkörpers 6 besitzt. Dadurch wird die Klappe 27 in ihrer in Fig. 2 dargestellten Geschlossenstellung eng von dem Rand 30 umgeben. Wenn die Klappe, sich aus dem Scharnier 28 herausbiegend, geöffnet ist, so wirkt dann ein von der Pumpeneinheit 10 ausgeübter Sog zunächst auf die dem Scharnier 28 und dem Rand 30 nahen Abschnitte der Klappe 27 ein, die damit von dort her zunehmend in den Ring 30 gesogen wird, bis sie schließlich die Durchlaßöffnungen vollständig abdeckt.

Um die Klappe 27 reinigen zu können, ist sie mit Hilfe des Ringflansches 29 auf den Ventilkörper 6 aufgeschoben. Der Ventilkörper 6 besitzt Vorsprünge 31, in die der Ringflansch 29 zur Befestigung elastisch einschnappt. Statt einer Reihe von Vorsprüngen 31 könnten auch mehrere Reihen vorgesehen werden, doch ist dies zur Erleichterung der Reinigung des vom Abzweigkanal 5 abnehmbaren Ventilkörpers 6 gar nicht besonders erwünscht. Fig. 2 zeigt auch, daß der Ventilkörper mit Längsrippen 32 versehen sein kann, doch ist dies nicht unbedingt erforderlich, und eine wenigstens im wesentlichen zylindrische Form ist bevorzugt, wenn auch andere Querschnittsformen denkbar wären. Auch ist klar, daß an Stelle von Rippen 31 am Ventilkörper 6 oder zusätzlich dazu eine Ringrippe 33 am freien Ende des Ringflansches 29 vorgesehen sein kann, die dann in eine Vertiefung des Ventilkörpers 6, z.B. hinter den Rippen 31, einrastet.

Im Rahmen der Erfindung versteht es sich, daß jede der oben angegebenen Maßnahmen zur Verhinderung des Eindringens von Milch in die Pumpeneinheit 10 für sich von erfinderischer Bedeutung ist, wenn auch ihre gemeinsame Verwirklichung ein Höchstmaß an Sicherheit gewährleistet.

## Patentansprüche

1. Muttermilchpumpe mit einem die Brust einer Frau aufnehmenden Brustkörper (1), der einen Abflußkanal (2) und eine von diesem über einen ersten Abzweigkanal (3, 4) abzweigende Pumpeneinheit (10) aufweist, wobei ein weiterer Abzweigkanal (5) über ein Rückschlagventil (6, 27) zu einem Milchsammler (8) führt, welches Ventil (6, 27) mit einem Ventilkörper (6) mit mindestens einer Durchlaßöffnung (26) für abgepumpte Muttermilch mit einer in Geschlossenstellung diese Öffnung (26) verschließenden Klappe (27) aus schmiegsamem Material versehen ist, die über eine Verbindungseinrichtung (28-33) mit dem Ventilkörper (6) verbindbar ist, **dadurch gekennzeichnet, daß** die Verbindungseinrichtung (28-33) ein am Rande der Stirnseite (25) angeordnetes Scharnier (28) für eine sich von diesem Scharnier (28) aus frei über wenigstens einen Teil der Stirnseite (25) erstreckende Klappe (27) und überdies einen den Ventilkörper (6) wenigstens teilweise umgebenden Befestigungsflansch (29) aufweist, wobei die Klappe (27) in der Geschlossenstellung an einer mit mindestens einer Durchtrittsöffnung (26) versehenen Stirnfläche (25) anliegt.

2. Muttermilchpumpe nach Anspruch 1, **dadurch gekennzeichnet, daß** der Befestigungsflansch (29), ebenso wie der Ventilkörper (6), im wesentlichen zylindrisch ausgebildet ist
und/oder
der Befestigungsflansch (29) bzw. der Ventilkörper (6) mindestens einen Vorsprung (31, 33) und/oder mindestens eine Vertiefung zur ineinandergreifenden Verbindung aufweist.

3. Muttermilchpumpe nach einem der vorhergehenden Ansprüche, **dadurch gekennzeichnet, daß** wenigstens eines der folgenden Merkmale vorgesehen ist:
a) es ist ein peripherer, das Scharnier (28) mit bildender Rand (30) vorgesehen, der die Klappe (27) in deren die Durchlaßöffnung (26) abschließenden Zustand mit endlicher Dicke eng umschließt;
b) die sich vom Scharnier (28) aus frei über die Stirnseite (25) erstreckende Klappe (27) deckt eine Mehrzahl von Durchlaßöffnungen (26) ab;
c) die Klappe (27) samt der das Scharnier (28) aufweisenden Verbindungseinrichtung (28-33) sind aus Kunststoff gefertigt.

4. Ventil für eine Muttermilchpumpe nach einem der vorhergehenden Ansprüche, mit einem Ventilkörper (6) mit mindestens einer Durchtrittsöffnung (26) für abgepumpte Muttermilch und einer diese Öffnung (26) beim Absaugen verschließenden Klappe (27) aus schmiegsamem Material, die über eine Verbindungseinrichtung (28-33) mit dem Ventilkörper (6) verbindbar ist, **dadurch gekennzeichnet, daß** die Verbindungseinrichtung (28-33) ein am Rande der Stirnseite (25) angeordnetes Scharnier (28) für eine sich von diesem Scharnier (28) aus frei über wenigstens einen Teil der Stirnseite (25) erstreckende Klappe (27) und überdies einen den Ventilkörper (6) wenigstens teilweise umgebenden Befestigungsflansch (29) aufweist, der im Gebrauchszustand die Stimfläche mit der mindestens einen Durchtrittsöffnung derart umgibt, daß die Klappe (27) in der Geschlossenstellung an einer mit mindestens einer Durchtrittsöffnung (26) versehenen Stirnfläche (25) anliegt.

5. Ventil nach Anspruch 4, **dadurch gekennzeichnet, daß** der Befestigungsflansch (29), ebenso wie der Ventilkörper (6), im wesentlichen zylindrisch ausgebildet ist, und/oder
daß der Befestigungsflansch (29) bzw. der Ventilkörper (6) mindestens einen Vorsprung (31, 33) und/oder mindestens eine Vertiefung zur ineinandergreifenden Verbindung aufweist.

6. Ventil nach einem der Ansprüche 4 oder 5, **dadurch gekennzeichnet, daß** wenigstens eines der folgenden Merkmale vorgesehen ist:
a) die sich vom Scharnier (28) aus frei über die Stirnseite (25) erstreckende Klappe (27) deckt eine Mehrzahl von Durchtrittsöffnungen (26) ab;
b) es ist ein peripherer, das Scharnier (28) mit bildender Rand (30) vorgesehen, der die Klappe (27) in deren die Durchlaßöffnung (26) abschließenden Zustand mit endlicher Dicke eng umschließt;
c) die Klappe (27) samt der das Scharnier (28) aufweisenden Verbindungseinrichtung (28-33) sind aus Kunststoff gefertigt.

## Claims

1. A breast pump comprising a breast body (1 ) for receiving the breast of a woman and having a drain channel (2) and a pump unit (10) branching from said drain channel (2) via a first branch channel (3, 4), a further branch channel (5) leading via a non-return valve (6, 27) to a milk collector (8), which valve (6, 27) is provided with a valve body (6) having at least one through-aperture (26) for pumped milk with a flap (27) of flexible material for closing this aperture (26) in the closed position, which flap (27) is connectable to the valve body (6) via a connecting arrangement (28-33), **characterised in that** the connecting arrangement (28-33) has a hinge (28) arranged at the edge of the end face (25) thereof for a flap (27) extending freely from this hinge (28) over at least a part of the end face (25) and, in addition, has a fixing flange (29) at least partially surrounding the valve body (6), the flap (27) abutting an end face (25) provided with at least one through-aperture (26).

2. A breast pump according to Claim 1, **characterised in that** the fixing flange (29), like the valve body (6), has a substantially cylindrical configuration, and/or the fixing flange (29) or the valve body (6) has at least one projection (31, 33) and/or at least one recess for interengaging connection.

3. A breast pump according to either of the preceding claims, **characterised in that** at least one of the following features is provided:
a) there is provided a peripheral edge (30) which partially forms the hinge (28) and which closely surrounds the flap (27) with finite thickness in the position of the flap (27) in which it closes the through-aperture (26);
b) the flap (27) extending freely from the hinge (28) over the end face (25) covers a plurality of through-apertures (26);
c) the flap (27), together with the connecting arrangement (28-33) including the hinge (28), are made of plastics material.

4. A valve for a breast pump according to any one of the preceding claims, comprising a valve body (6) having at least one through-aperture (26) for pumped milk and a flap (27) of flexible material for closing said through-aperture (26) during suction, which flap (27) is connectable to the valve body (6) via a connecting arrangement (28-33), **characterised in that** the connecting arrangement (28-33) has a hinge (28) arranged at the edge of the end face (25) thereof for a flap (27) extending freely from this hinge (28) over at least a part of the end face (25) and, in addition, has a fixing flange (29) at least partially surrounding the valve body (6), which fixing flange (29) surrounds the end face having the at least one through-aperture in the operating state in such a way that the flap (27) abuts an end face (25) provided with at least one through-aperture (26) in the closed position.

5. A valve according to Claim 4, **characterised in that** the fixing flange (29), like the valve body (6), has a substantially cylindrical configuration, and/or the fixing flange (29) or the valve body (6) has at least one projection (31, 33) and/or at least one recess for interengaging connection.

6. A valve according to either of claims 4 or 5, **characterised in that** at least one of the following features is provided:
a) the flap (27) extending freely from the hinge (28) over the end face (25) covers a plurality of through-apertures (26);
b) there is provided a peripheral edge (30) which partially forms the hinge (28) and which closely surrounds the flap (27) with finite thickness in the position of the flap (27) in which it closes the through-aperture (26);
c) the flap (27), together with the connecting arrangement (28-33) including the hinge (28), are made of plastics material.

## Revendications

1. Tire-lait muni d'un corps pour sein (1) recevant le sein d'une femme, présentant un canal d'évacuation (2) et une unité de pompage (10) se ramifiant depuis celui-ci par l'intermédiaire d'un premier canal de ramification (3, 4), un autre canal de ramification (5) menant, par un clapet anti-retour (6, 27), à un collecteur de lait (8), le clapet (6, 27) étant muni d'un opercule (6) portant au moins une ouverture de passage (26) pour le lait maternel ayant été pompé, avec un clapet (27) fermant cette ouverture (26) lorsqu'il se trouve à la position fermée et étant réalisé en un matériau pouvant épouser les formes, susceptible d'être relié à l'opercule (6) par l'intermédiaire d'un dispositif de liaison (28-33), **caractérisé en ce que** le dispositif de liaison (28-33) présente une charnière (28), disposée sur le bord de la face frontale (25), pour un clapet (27) s'étendant depuis cette charnière (28), librement, sur au moins une partie de la face frontale (25), et, en plus de cela, présente une bride de fixation (29) entourant au moins partiellement l'opercule (6), le clapet (27), à la position fermée, venant en appui sur une face frontale (25) munie d'au moins une ouverture de passage (26).

2. Tire-lait selon la revendication 1, **caractérisé en ce que** la bride de fixation (29), tout comme l'opercule (6), est de forme sensiblement cylindrique
et/ou
la bride de fixation (29), respectivement l'opercule (6), présente(nt) au moins une saille (31, 33) et/ou au moins un creusement, pour obtenir une liaison par engagement l'un dans l'autre.

3. Tire-lait selon l'une des revendications précédentes, **caractérisé en ce qu'**est prévue au moins l'une des caractéristiques suivantes :
a) il est prévu un bord (30) périphérique formant conjointement la charnière (28), bord entourant étroitement, avec une épaisseur finie, le clapet (27) lorsqu'il est à son état fermant l'ouverture de passage (26) ;
b) le clapet (27), s'étendant depuis la charnière (28), librement, sur la face frontale (25), recouvre une pluralité d'ouvertures de passage ;
c) le clapet (27), avec le dispositif de liaison (28-33) présentant la charnière (28), sont fabriqués en matière synthétique.

4. Soupape pour un tire-lait selon l'une des revendications précédentes, avec un opercule (6), comportant au moins une ouverture de passage (26) pour du lait maternel aspiré, et un clapet (29), fermant cette ouverture (26) lors de l'aspiration et réalisé en un matériau susceptible d'épouser les formes, le clapet pouvant être relié à l'opercule (6) par l'intermédiaire d'un dispositif de liaison (28-33), **caractérisé en ce que** le dispositif de liaison (28-33) présente une charnière (28) disposée sur le bord de la face frontale (25), pour un clapet (27) s'étendant de cette charnière (28), librement, sur au moins une partie de la face frontale (25) et, en plus de cela, le dispositif de liaison présente une bride de fixation (29) entourant au moins partiellement l'opercule (6), bride qui, à l'état d'utilisation, entoure la face frontale par au moins une ouverture de passage, **en ce que** le clapet (27), à la position fermée, vient en appui sur une face frontale (25) munie d'au moins une ouverture de passage (26).

5. Soupape selon la revendication 1, **caractérisée en ce que** la bride de fixation (29), tout comme l'opercule (6), sont de configuration sensiblement cylindrique, et/ou
**en ce que** la bride de fixation (29) ou l'opercule (6) présente(nt) au moins une saillie (31, 33) et/ou au moins une cavité, pour obtenir une liaison avec engagement l'un dans l'autre.

6. Soupape selon l'une des revendications 4 ou 5, **caractérisée en ce qu'**est prévue au moins l'une des caractéristiques suivantes :
a) le clapet (27), s'étendant de la charnière (28), librement, sur la face frontale (25), recouvre une pluralité d'ouvertures de passage (26) ;
b) il est prévu un bord (30) périphérique formant conjointement la charnière (28), bord qui entoure étroitement, avec une épaisseur finie, le clapet (27) lorsqu'il est à son état obturant l'ouverture de passage (26) ;
c) le clapet (27), avec le dispositif de liaison (28-33) présentant la charnière (28), sont fabriqués en matière synthétique.
